# EUROPEAN PATENT SPECIFICATION

(11) **EP 1 532 112 B1**
(45) Date of publication and mention of the grant of the patent: **26.12.2007**
(21) Application number: 03765215.3
(22) Date of filing: 07.07.2003
(51) Int. Cl.: C07D 213/74

(54) **PROCESS FOR THE PREPARATION OF MODIFICATION I OF N-(1-METHYLETHYLAMINOCARBONYL)-4-(3-METHYLPHENYLAMINO)-3-PYRIDINESULFONAMIDE**
VERFAHREN ZUR HERSTELLUNG VON MODIFIKATION I VON N-(1-METHYLETHYLAMINOCARBONYL)-4-(3-METHYLPHENYLAMINO)-3 PYRIDINSULFONAMID
PROCEDE DE PREPARATION DE LA MODIFICATION I DE N-(1-METHYLETHYLAMINOCARBONYL)-4-(3-METHYLPHENYLAMINO)-3-PYRIDINESULFONAMIDE

(30) Priority: 19.07.2002 HR 20020603
(43) Date of publication of application: 25.05.2005
(73) Proprietor: PLIVA HRVATSKA d.o.o., 10000 Zagreb (HR)
(72) Inventor: FILIC, Darko, 10000 Zagreb (HR); DUMIC, Miljenko, 10000 Zagreb (HR); DANILOVSKI, Aleksandar, 51000 Rijeka (HR); KLEPIC, Bozena, 10450 Jastrebarsko (HR); FISTRIC, Ines, 10000 Zagreb (HR); MARINKOVIC, Marina, 10360 Sesvete (HR); HORVAT-MIKULCIC, Jasna, 10000 Zagreb (HR)
(74) Representative: McLeish, Nicholas Alistair Maxwell
(86) International application number: PCT/HR2003/000036
(87) International publication number: WO 2004/009554

(56) References cited:
- WO-A-00/20395
- WO-A-01/10441
- US-A- 4 743 693
- US-A- 6 166 045
- ROLLINGER J M ET AL: "Crystal forms of torasemide: new insights" EUROPEAN JOURNAL OF PHARMACEUTICS AND BIOPHARMACEUTICS, ELSEVIER SCIENCE PUBLISHERS B.V., AMSTERDAM, NL, vol. 53, no. 1, January 2002 (2002-01), pages 75-86, XP004331334 ISSN: 0939-6411

## Description

The present invention relates to the preparation of modification I of *N*-(1-methylethyl aminocarbonyl)-4-(3-methylphenylamino)-3-pyridinesulfonamide (in the further text of the application designated by its generic name "torasemide").

Torasemide is a new potent diuretic in the class of so-called "loop" diuretics and is described in DE patent 25 16 025 (Example 71) as 3-isopropylcarbamylsulfonamide-4-(3'-methyl)-phenylamino pyridine. Structurally, it is completely different from diuretics of the same class such as e.g. furosemide, bumetanide and azosemide. Besides diuretic properties it also possesses antihypertensive ones.

As a diuretic of Henle's loop it is interesting as an agent for preventing heart or heart tissue damages caused by metabolic or ionic abnormalities associated with ischemia, in the treatment of thrombosis, angina pectoris, asthma, hypertension, nephroedema, pulmonary edema, primary and secondary aldosteronism, Bartter's syndrome, tumours, glaucoma, decrease of intraocular pressure, acute or chronic bronchitis, in the treatment of cerebral edema caused by trauma, ischemia, concussion of the brain, metastases or epileptic attacks and in the treatment of nasal infections caused by allergens.

Hitherto, some crystal modifications of torasemide have been known: modification I *[Acta Cryst.* B34 (1978), 1304-1310], modification II *[Acta Cryst.* B34 (1978), 2659-2662], modification III (US patent 6,166,045), modification N (US patent 6,399,637), modification V (PLIVA; PCT/WO 01/87841), modification V (TEVA; PCT/WO 01/10441), as well as an amorphous modification of PLIVA (PCT/WO 01/70694), an amorphous modification of TEVA (PCT/WO 01/10441) and Dupont 2 solvate adducts (PCT/WO 01/10441). Crystal modifications I, II and N differ in single cell parameters, which is confirmed by X-ray diffraction on their monocrystals. Modification I with melting point 169°C *[*Acta Cryst. B34 (1978), 1304-1310] and modification N with melting point 165 °C [US patent 6,399,637; Croat. Chem. Acta 74 (2001) 103-120] crystallize in the monoclinic space group P2₁/c (prisms), while modification II with melting point 162 °C crystallizes in the monoclinic space group P2/n (foils) [Acta Cyst. B34 (1978), 2659-2662].

It is known that modification I of torasemide and modification II of torasemide crystallize simultaneously when a torasemide solution in a solvent mixture petroleum ether/ethanol slowly evaporates [Acta Cryst. B34 (1978), 1304-1310]. Such a manner of preparation, however, wherein both modifications crystallize from the same solvent mixture and hence must be separated with regard to their macroscopic crystal form, is certainly not suitable for large-scale production.

Further, in the patent application PCT/WO 01/10441 there is described the preparation of modification I of torasemide by recrystallization from methanol of modification II of torasemide or of a mixture of modifications I and II of torasemide or of modification V (TEVA) of torasemide or of Dupont Form 2 solvate adducts of torasemide as well as of their mixtures.

Contrary to the above, in the text of patents US 4,743,693; US reissue 34,580; US 4,822,807; US reissue 34,672; US 5,914,336 and US 6,166,045 it is stated that by recrystallization from solvents always modification II of torasemide is formed. In addition, it is known that by heating torasemide in most solvents its irreversibile decomposition takes place (US 4,743,693; US reissue 34,580; US 4,822,807; US reissue 34,672), whereby the content of accompanying impurities is increased. In view of these statements, recrystallization is not suitable method for the preparation of modification I of torasemide.

The patents US 4,743,693 and US reissue 34,580 protect a process for the preparation of modification I of torasemide, wherein a suspension of modification II of torasemide prepared according to patent DE 25 16 025 in water is stirred under the addition of a catalytic amount of modification I of torasemide (0.1 %) at a temperature from room temperature to 90 °C within 3 hours to 14 days. It is stated that no decomposition of torasemide takes place. The patent application PCT/WO01/10441 discloses the preparation of modification I of torasemide by stirring modification II of torasemide or a mixture of modifications I and II of torasemide in a solvent mixture containing acetonitrile under reflux, or in a mixtureof DMSO : acetonitrile at a temperature from 20°C to 30 °C within 30 to 45 minutes or in a solvent mixture of water: acetonitrile at a temperature from 40 °C to 60 °C over a period of more than 45 minutes. In the same patent application also the preparation of modification I of torasemide by stirring Dupont Form 2 of torasemide in water at pH 5 and at room temperature or by stirring modification II of torasemide in ethanol or dimethylformamide also at room temperature is disclosed.

From the text of patents US 4,743, 693;US reissue 34,580; US 4,822,807;US reissue 34,672 ; US 5,914, 336 and US 6,166, 045 it is evident that by the preparation and conventional purification of crude torasemide either by precipitation or by recrystallization from solvents the modification II of torasemide is obtained.

Rollinger et al.* (European Journal of Pharmaceutics and Biopharmaceutics, vol 53, no1, p75-86) discloses the cristallisation of torasemide I from a hot saturated solution of methanol, ethanol, and propanol by cooling. The WO 00/20395 A relates to the production of torasemide III.

In our further investigations in the torasemide field we have surprisingly found a new, hitherto not known process for the preparation of modification I of torasemide.

The new process for the preparation of modification I of torasemide comprises the preparation of modification I of torasemide by directly acidifying an alkaline extract of the original reaction mixture of the last phase in the synthesis of torasemide. The preparation process of the invention enables a more effective, more rapid and economically more acceptable method for the preparation of modification I of torasemide.

According to the process of the invention, for the preparation of the alkaline extract of the original reaction mixture of last phase in the synthesis of torasemide there can be used water solutions of alkaline hydroxides such as solutions of lithium, sodium and potassium hydroxide as well as water solutions of alkaline carbonates such as solutions of sodium and potassium carbonate.

According to the present process, the acidifying of the alkaline extract of the original reaction mixture of the last phase in the synthesis of torasemide can be performed with inorganic acids such as hydrochloric, sulfuric, phosphoric and nitric acids and with organic acids such as formic, acetic, propionic, oxalic, tartaric, methanesulfonic and p-toluenesulfonic acids and carbon dioxide.

The acidifying of the alkaline extract of the original reaction mixture of the last phase in the synthesis of torasemide is performed by a continuous addition of the acid at room temperature or about it, preferably under stirring at a stirrer rate from 10 to 300 r/min within 5 minutes to 24 hours until a pH from about 8.5 to about 5.0, most preferably from about 7.5 to about 7.0 is reached, which depends on the acid/base concentration as well as on the batch size, the volume, the construction and the geometry of the reactor. During the addition of the acid it is not necessary to avoid local high concentrations of the acid.

After reaching the desired pH, the suspension is preferably stirred for another 10 to 240 minutes at a temperature from about 0 to about 50 °C, most preferably at room temperature, in order to complete the crystallization. The crystals are separated from the water medium in a usual manner such as filtration, and then dried down to a low moistur content, most preferably below 0.5%.

It has been found that by using of the process of the present invention no decomposition of torasemide occurs, modification I of torasemide contains the remaining solvents within pharmacopeic limits and the impurities that are possibly present in the alkaline extract of the original reaction mixture of the last phase in the synthesis of torasemide pass into the bases, which means that a chemically pure modificatio I of torasemide is obtained.

Moreover, it has been found that the stable modification I of torasemide prepared according to the process of the present invention is obtained in a"free flow" form, i. e. suitable for the preparation of pharmaceutical forms such as tablets, capsules or injections.

### Example 1

The original reaction mixture of the last phase in the synthesis of torasemide (starting from 100 g of 4-hydroxy-3-pyridinesulfonic acid) was treated with an about 10 % aqueous solution of sodium hydroxide. To the alkaline solution an about 10 % aqueous acetic acid solution was continuously added within 30 minutes at room temperature under a stirring rate of about 50 r/min to reach pH 7.0. Then the obtained suspension was stirred for further 30 minutes at the same temperature. The crystals were sucked off and washed with water, whereupon, after drying in a vacuum dryer to a constant weight, 100 g of modification I of torasemide were obtained.

Content according to HPLC 99.2 %, moisture < 0.2 %

The IR spectrum and the X-ray powder pattern of the thus obtained sample of modification I of torasemide corresponded to the IR spectrum and the X-ray powder pattern of an authentic sample of modification I of torasemide obtained according to Acta Cryst. B34 (1978), 1304-1310.

### Example 2

The original reaction mixture of the last phase in the synthesis of torasemide (starting from 10.0 kg of 4-hydroxy-3-pyridinesulfonic acid) was treated with an about 2.5 % aqueous solution of sodium hydroxide. To the solution an about 5 % aqueous hydrochloric acid solution was added within 60 minutes at room temperature under a stirring rate of about 200 r/min to reach pH 7.1-7.2. Then the obtained suspension was stirred for further 60 minutes at the same temperature. The crystals were sucked off and washed with water, whereupon, after drying in a vacuum dryer up to a constant weight, 11.0 kg of a stable modification I of torasemide were obtained.

Content according to HPLC 99.1 %, moisture 0.3 %

The IR spectrum and the X-ray powder pattern of the thus obtained sample of modification I of torasemide corresponded to the IR spectrum and the X-ray powder pattern of an authentic sample of modification I of torasemide obtained according to Acta Cryst. B34 (1978), 1304-1310.

## Claims

1. Process for the preparation of modification I of torasemide, **characterized in that** an alkaline extract of the original reaction mixture of the last phase in the synthesis of torasemide is subjected to controlled acidifying with inorganic or organic acid by continuous addition of said acid at room temperature or about it.

2. Process for the preparation of modification I of torasemide according to claim 1, **characterized in that** the modification I of torasemide is chemically pure.

3. Process for the preparation of modification I of torasemide according to claim 1, **characterized in that** the modification I of torasemide contains less than 0.5 % of water.

4. Process for the preparation of modification I of torasemide according to claim 1, **characterized in that** the modification I contains remaining solvents within pharmacopeic limits.

5. Process for the preparation of modification I of torasemide according to claim 1, **characterized in that** for the preparation of the alkaline extract of the original reaction mixture of the last phase in the synthesis of torasemide water solutions of lithium, sodium and potassium hydroxide and water solutions of sodium and potassium carbonate are used.

6. Process for the preparation of modification I of torasemide according to claim 1, **characterized in that** for acidifying the alkaline extract of the original reaction mixture of the last phase in the synthesis of torasemide inorganic acids such as hydrochloric, sulfuric, phosphoric and nitric acids or organic acids such as formic, acetic, propionic, oxalic, tartaric, methanesulfonic orp-toluenesulfonic acid are used.

7. Process for the preparation of modification I of torasemide according to claim 1, **characterized in that** for acidifying the alkaline extract of the original reaction mixture of the last phase in the synthesis of torasemide carbon dioxide is used.

8. Process for the preparation of modification I of torasemide according to claim 1, **characterized in that** the acidifying is carried out up to a pH from about 8.5 to about 5.0.

9. Process for the preparation of modification I of torasemide according to claim 8, **characterized in that** the acidifying is carried out up to a pH from about 7.5 to about 7.0.

10. Process for the preparation of modification I of torasemide according to claim 1, **characterized in that** the acidifying is carried out at a stirrer rate from 10 r/min to 300 r/min.

11. Process for the preparation of modification I of torasemide according to claim 1, **characterized in that** the acidifying is carried out within 5 minutes to 24 hours.

12. Process for the preparation of modification I of torasemide according to claim 1, **characterized in that** the acidifying is carried out without avoiding high local acid concentrations.

13. Process for the preparation of modification I of torasemide according to claim 1, **characterized in that** the suspension obtained after acidifying and reaching the desired pH is stirred from 10 minutes to 240 minutes.

14. Process for the preparation of modification I of torasemide according to claim 13, **characterized in that** the suspension obtained after acidifying and reaching the desired pH is stirred at a temperature from 0 °C to 50°C.

15. Process for the preparation of modification I of torasemide according to claim 14, **characterized in that** the suspension obtained after acidifying and reaching the desired pH is stirred at room temperature.

## Patentansprüche

1. Verfahren zur Herstellung von Modifikation I von Torasemid, **dadurch gekennzeichnet, dass** ein alkalischer Extrakt des ursprünglichen Reaktionsgemischs aus der letzten Phase der Synthese von Torasemid kontrolliertem Ansäuern mit anorganischer oder organischer Säure unterzogen wird, indem die Säure kontinuierlich bei oder ungefähr bei Raumtemperatur zugegeben wird.

2. Verfahren zur Herstellung von Modifikation I von Torasemid gemäß Anspruch 1, **dadurch gekennzeichnet, dass** die Modifikation I von Torasemid chemisch rein ist.

3. Verfahren zur Herstellung von Modifikation I von Torasemid gemäß Anspruch 1, **dadurch gekennzeichnet, dass** die Modifikation I von Torasemid weniger als 0,5% Wasser enthält.

4. Verfahren zur Herstellung von Modifikation I von Torasemid gemäß Anspruch 1, **dadurch gekennzeichnet, dass** die Modifikation I Lösungsmittelreste innerhalb Pharmakopöe-Grenzen enthält.

5. Verfahren zur Herstellung von Modifikation I von Torasemid gemäß Anspruch 1, **dadurch gekennzeichnet, dass** zur Herstellung des alkalischen Extrakts des ursprünglichen Reaktionsgemischs aus der letzten Phase der Synthese von Torasemid wässrige Lösungen von Lithium-, Natrium- und Kaliumhydroxid und wässrige Lösungen von Natrium- und Kaliumcarbonat verwendet werden.

6. Verfahren zur Herstellung von Modifikation I von Torasemid gemäß Anspruch 1, **dadurch gekennzeichnet, dass** zum Ansäuern des alkalischen Extrakts des ursprünglichen Reaktionsgemischs aus der letzten Phase der Synthese von Torasemid anorganische Säuren wie etwa Chlorwasserstoff-, Schwefel-, Phosphor- und Salpetersäuren oder organische Säuren wie etwa Ameisen-, Essig-, Propion-, Oxal-, Wein-, Methansulfon- oder p-Toluolsulfonsäure verwendet werden.

7. Verfahren zur Herstellung von Modifikation I von Torasemid gemäß Anspruch 1, **dadurch gekennzeichnet, dass** zum Ansäuern des alkalischen Extrakts des ursprünglichen Reaktionsgemischs aus der letzten Phase der Synthese von Torasemid Kohlendioxid verwendet wird.

8. Verfahren zur Herstellung von Modifikation I von Torasemid gemäß Anspruch 1, **dadurch gekennzeichnet, dass** das Ansäuern bis zu einem pH-Wert von etwa 8,5 bis etwa 5,0 durchgeführt wird.

9. Verfahren zur Herstellung von Modifikation I von Torasemid gemäß Anspruch 8, **dadurch gekennzeichnet, dass** das Ansäuern bis zu einem pH-Wert von etwa 7,5 bis etwa 7,0 durchgeführt wird.

10. Verfahren zur Herstellung von Modifikation I von Torasemid gemäß Anspruch 1, **dadurch gekennzeichnet, dass** das Ansäuern bei einer Rührergeschwindigkeit von 10 U/min bis 300 U/min durchgeführt wird.

11. Verfahren zur Herstellung von Modifikation I von Torasemid gemäß Anspruch 1, **dadurch gekennzeichnet, dass** das Ansäuern innerhalb von 5 Minuten bis 24 Stunden durchgeführt wird.

12. Verfahren zur Herstellung von Modifikation I von Torasemid gemäß Anspruch 1, **dadurch gekennzeichnet, dass** das Ansäuern durchgeführt wird, ohne hohe lokale Säurekonzentrationen zu vermeiden.

13. Verfahren zur Herstellung von Modifikation I von Torasemid gemäß Anspruch 1, **dadurch gekennzeichnet, dass** die nach Ansäuern und Erreichen des gewünschten pH-Wertes erhaltene Suspension für 10 Minuten bis 240 Minuten gerührt wird.

14. Verfahren zur Herstellung von Modifikation I von Torasemid gemäß Anspruch 13, **dadurch gekennzeichnet, dass** die nach Ansäuern und Erreichen des gewünschten pH-Wertes erhaltene Suspension bei einer Temperatur von 0°C bis 50°C gerührt wird.

15. Verfahren zur Herstellung von Modifikation I von Torasemid gemäß Anspruch 14, **dadurch gekennzeichnet, dass** die nach Ansäuern und Erreichen des gewünschten pH-Wertes erhaltene Suspension bei Raumtemperatur gerührt wird.

## Revendications

1. Procédé pour la préparation de la variété I du torasémide, **caractérisé en ce qu'**un extrait alcalin du mélange réactionnel d'origine de la dernière phase de la synthèse du torasémide est soumis à une acidification ménagée avec un acide organique ou minéral par addition continue dudit acide à la température ambiante ou une température voisine de la température ambiante.

2. Procédé pour la préparation de la variété I du torasémide selon la revendication 1, **caractérisé en ce que** la variété I du torasémide est chimiquement pure.

3. Procédé pour la préparation de la variété I du torasémide selon la revendication 1, **caractérisé en ce que** la variété I du torasémide contient moins de 0,5 % d'eau.

4. Procédé pour la préparation de la variété I du torasémide selon la revendication 1, **caractérisé en ce que** la variété I contient des solvants résiduels dans les limites admises par la pharmacopée.

5. Procédé pour la préparation de la variété I du torasémide selon la revendication 1, **caractérisé en ce que**, pour la préparation de l'extrait alcalin du mélange réactionnel d'origine de la dernière phase de la synthèse du torasémide, on utilise des solutions aqueuses d'hydroxyde de lithium, sodium et potassium et des solutions aqueuses de carbonate de sodium et potassium.

6. Procédé pour la préparation de la variété I du torasémide selon la revendication 1, **caractérisé en ce que**, pour acidifier l'extrait alcalin du mélange réactionnel d'origine de la dernière phase de la synthèse du torasémide, on utilise des acides minéraux tels que les acides chlorhydrique, sulfurique, phosphorique et nitrique ou des acides organiques tels que l'acide formique, acétique, propionique, oxalique, tartrique, méthanesulfonique ou p-toluènesulfonique.

7. Procédé pour la préparation de la variété I du torasémide selon la revendication 1, **caractérisé en ce que**, pour acidifier l'extrait alcalin du mélange réactionnel d'origine de la dernière phase de la synthèse du torasémide, on utilise du dioxyde de carbone.

8. Procédé pour la préparation de la variété I du torasémide selon la revendication 1, **caractérisé en ce que** l'acidification est effectuée jusqu'à un pH d'environ 8,5 à environ 5,0.

9. Procédé pour la préparation de la variété I du torasémide selon la revendication 8, **caractérisé en ce que** l'acidification est effectuée jusqu'à un pH d'environ 7,5 à environ 7,0.

10. Procédé pour la préparation de la variété I du torasémide selon la revendication 1, **caractérisé en ce que** l'acidification est effectuée à une vitesse d'agitateur de 10 tr/min à 300 tr/min.

11. Procédé pour la préparation de la variété I du torasémide selon la revendication 1, **caractérisé en ce que** l'acidification est effectuée en 5 minutes à 24 heures.

12. Procédé pour la préparation de la variété I du torasémide selon la revendication 1, **caractérisé en ce que** l'acidification est effectuée sans éviter de fortes concentrations locales d'acide.

13. Procédé pour la préparation de la variété I du torasémide selon la revendication 1, **caractérisé en ce que** la suspension obtenue après acidification et obtention du pH désiré est agitée pendant 10 minutes à 240 minutes.

14. Procédé pour la préparation de la variété I du torasémide selon la revendication 13, **caractérisé en ce que** la suspension obtenue après acidification et obtention du pH désiré est agitée à une température de 0°C à 50°C.

15. Procédé pour la préparation de la variété I du torasémide selon la revendication 14, **caractérisé en ce que** la suspension obtenue après acidification et obtention du pH désiré est agitée à la température ambiante.
